# EUROPEAN PATENT APPLICATION

(11) **EP 2 327 777 A1**
(43) Date of publication of application: **01.06.2011**
(21) Application number: 09808453.6
(22) Date of filing: 07.08.2009
(51) Int. Cl.: C12N 11/08, C12N 11/10, A61K 38/00, A61K 38/43, A61K 47/48

(54) **METHOD FOR IMMOBILISING BIOLOGICALLY ACTIVE SUBSTANCES ON POLYMER CARRIERS (VARIANTS) AND CONJUGATES PRODUCED BY SAID METHOD**

(30) Priority: 18.08.2008 RU 2008133940
(71) Applicant: Scientific Future Management SFM, 630056 Novosibirsk (RU)
(72) Inventor: BEKAREV Andrei Alexandrovich, Novosibirsk 630090 (RU); ARTAMONOV Andrei Vladimirovich, Novosibirsk 630005 (RU); VERESCHAGIN Evgeny Ivanovich, Novosibirsk 630083 (RU)
(74) Representative: HOFFMANN EITLE
(86) International application number: PCT/RU2009/000396
(87) International publication number: WO 2010/021570

(57) **Abstract**

The invention relates to medicine, biology, pharmacology, in particular to a method for immobilizing a biologically active substance on a pharmacologically acceptable polymer carrier. The inventive method involves dissolving a biologically active substance in a polymer carrier aqueous solution, the concentration of which is greater than 0%, up to a saturated solution concentration and irradiating the reaction mixture by ionizing radiation. In order to protect the biologically active substance from being destroyed by ionizing radiation, the reaction mixture can be frozen at a temperature ranging from -20°C to -140°C prior to be irradiated. While being immobilized according to the inventive method, the biologically active substance is covalently linked to the polymer. The advantage of the inventive method consists in that it makes possible to immobilize on the polymer carrier those water-soluble biologically active substances which are unstable, i.e., which are destroyed totally or in part when immobilized by the other methods and to increase the portion of the preserved biologically active substance, when the biologically active substance is not totally destroyed during immobilization. The conjugate as a biologically active substance carrier is **characterized in that** the biologically active substance is selected from a group comprising proinsulin, tyrosyl-2-alanyl-glyceryl-phenylalanyl-leucyl-arginine diacetate, alcalase, hyaluronidase, somatotropin and in that the carrier is in the form of a pharmacologically acceptable water-soluble polymer which is selected from a group comprising polyethyleneoxide, polyvinylpirrolidone, dextran, pluronic and acrilamide, wherein a biologically active substance molecule is covalently linked to at least one polymer molecule.

## Description

The invention relates to medicine, biology, and pharmacology.

### Description of the Prior Art

The advanced tools of experimental and practical medicine include medications, frequently of a protein origin, that have an evidenced high efficiency with respect to many pathologies (hereinafter, they are referred to as biologically active substances, or BASs). They exert an effect on both the receptor system of a cell and via direct action on pathologic carriers, such as thrombi, protein detritus, and cellular membrane debris.

While having a high efficiency, these BASs have some undesirable properties. Protein medications can cause allergic reactions. Some BASs are rapidly destroyed in the bloodstream or while being stored. However, their potential for use is attractive to clinician physicians so as to make evident the necessity for modifying BAS molecules in a way to provide the safety of the medication together with saving the pharmacological efficiency thereof.

A conventional approach to this problem involves immobilization of a BAS on a water-soluble carrier polymer (polyethylene oxide, polyacrylamide, dextran, and so on). Immobilization increases the lifetime of the medication in bloodstream, thereby extending the duration of exposure thereof; decreases the immunogenicity of the medication; and provides the full activity thereof in a dosage form during long-term storage.

There are various methods for immobilizing a BAS on a polymer.

Chemical ways are known for BAS immobilization on a polymer. Patent WO 0044785, for example, describes a granulocyte colony stimulating factor (gCSF) conjugate with polyethylene oxide having a molecular mass between 5,000 and 30,000 Da obtained via synthesizing a peptide bond with use of reactive esters. A similar method was used to prepare conjugates described in patent CN1966528, but therein two polyethylene oxide molecules having a molecular mass of 5,000 to 30,000 Da linked to each other were used as carriers. The medication is used for injective administration.

The method provides covalent linkage between the BAS and polymer; however, as a result of use of rather aggressive chemicals, a high percentage of the BAS is destroyed during immobilization. This procedure involves a set of consecutive chemical reactions, which complicates the synthesis of the final product and requires additional technological steps for purifying the medication on account of the toxicity of the reagents used in the process for a living body. The final costs of the pharmacological agent increase considerably.

There is an enzyme-assisted method for immobilizing BASs on polymers. Patent WO2005055946, for example, describes a conjugate where gCSF and polyethylene oxide (polypropylene oxide) are linked to each other via sialic acid. Transferase is used to synthesize such the conjugates.

This method also provides covalent linkage between a BAS and a polymer, but it involves many steps, too, thereby considerably complicating the synthesis of the final product, and requires additional technological steps for purifying the medication.

Along with the aforementioned chemical method and enzyme-assisted method, electron-beam technologies are used to produce BAS - polymer conjugates.

A method is known for immobilization of amphotericin B on dextran (a water-soluble polymer), wherein an accelerated electron beam or γ-rays in a dose of 1 to 5 Mrad are used to irradiate dextran to reach the activation thereof (see RU2297246). Then, a BAS is inserted into the activated polymer. Dextran having a molecular mass of 30 to 60 kDa in concentrations of 1 to 10% is used in this method. The ratio of amphotericin B to dextran in the reaction mixture is I to 800. The yield of the therapeutic composition is 99%.

When immobilization is performed in this way, unstable BASs are not subjected to destruction, but this method does not provide covalent cross-linkage between the BAs and polymer; therefore, the positive effects of immobilization are weakened.

A method is known for immobilization of protosubtilin on polyethylene oxide, wherein protosubtilin in a solution of polyethylene oxide having a molecular mass of 1500 to 4000 Da is irradiated with bremsstrahlung γ-rays in doses of 0.5 to 1.0 Mrad (see RU2270861, which is the most pertinent art). Polyethylene oxide is added to the protosubtilin solution to reach a final concentration of 5 to 10% by weight in a ratio of (3-8):1. The yield of the final product amounts to 95-98%. The medication can be used parenterally.

In carrying out this method, we discovered the following drawback thereof: some BASs (polypeptides, proinsulin, insulin, somatotropic hormone, interferon-alpha, and others) were destroyed practically completely during immobilization under an impact of ionizing radiation in a dose of 0.5 to 1 MRad. Lower exposure doses did not provide BAS immobilization and conjugate formation.

### Disclosure of the Invention

The first variation of the method for immobilization of a biologically active substance (BAS) on a pharmacologically acceptable carrier polymer consists in that the BAS is dissolved in an aqueous solution of the carrier polymer having a concentration ranging from above 10% to a saturated solution and then the reaction mixture is exposed to ionizing radiation. In order to improve the resistance of the BAS to destruction by ionizing radiation, the reaction mixture may be frozen at temperature ranging from -20°C to -140°C before being irradiated.

Useful ionizing radiations are accelerated electron beams or bremsstrahlung γ-rays in doses of 0.5 to 5 Mrad.

Specifically, this method can serve to immobilize alcalase, insulin, tyrosyl-2-alanyl-glycyl-phenylalanyl-leucyl-arginine diacetate, granulocyte colony stimulating factor (gCSF), hyaluronidase, proinsulin, somatotropin, and other BASs on a carrier polymer.

Specifically useful water-soluble carrier polymers are polyethylene oxides, dextrans, polyvinylpyrrolidone, acrylamide, and some polymer surfactants, for example, Pluronic, and some others.

Further in the text, particular embodiments of this method will be given together with examples illustrating the preparation of alcalase conjugates with polyethylene oxide, insulin conjugates with polyethylene oxide, tyrosyl-2-alanyl-glycyl-phenylalanyl-leucyl-arginine diacetate conjugates with polyvinylpyrrolidone, gCSF conjugates with polyethylene oxide, hyaluronidase conjugates with dextran, and insulin conjugates with Pluronic.

During immobilization carried out by the present method, a BAS is covalently linked to a polymer.

The technical result of the method according to variation no. 1 is the provision of immobilization on a carrier polymer of an unstable water-soluble BASs, that is, a BAS that is destroyed when immobilized by the other methods, specifically, under the conditions created by the closest-art method where ionizing radiation is used in doses of 0.5 to 1 Mrad and polymer concentrations are 5 to 10%; in cases where a BAS is not destroyed completely during immobilization, the technical result consists of an increased percent survival of the BAS.

This technical result under the conditions of the method of the present invention is provided by polymer concentrations ranging from above 10% to a saturated solution.

The second variation of the method for immobilization of a BAS on a pharmacologically acceptable carrier polymer consists of that the BAS is dissolved in an aqueous solution of the carrier polymer and then the mixture is frozen to temperature ranging from -20°C to -140°C, afterwards being exposed to ionizing radiation.

A water-soluble pharmacologically acceptable polymer carrier is used in concentrations ranging from above 0% to a saturated solution.

Useful ionizing radiations are accelerated electron beams or bremsstrahlung γ-rays in doses of 0.5 to 5 Mrad.

Specifically, this method is useful to immobilize alcalase, insulin, tyrosyl-2-alanyl-glycyl-phenylalanyl-leucyl-arginine diacetate, granulocyte colony stimulating factor (gCSF), hyaluronidase, proinsulin, somatotropin, and other BASs on a carrier polymer.

Specifically useful water-soluble carrier polymers are polyethylene oxides, dextrans, polyvinylpyrrolidone, acrylamide, some polymer surfactants, for example, Pluronic, and some others.

Further in the text, particular embodiments of this method will be given together with examples illustrating the preparation of tyrosyl-2-alanyl-glycyl-phenylalanyl-leucyl-arginine diacetate conjugates with polyvinylpyrrolidone, insulin conjugates with polyethylene oxide, somatotropin conjugates with dextran, gCSF conjugates with polyethylene oxide, hyaluronidase conjugates with dextran, and insulin conjugates with Pluronic.

During immobilization carried out by the present method, a BAS is covalently linked to a polymer.

The technical result of the method according to variation no. 2 is the same as described above for variation no. 1, namely: the provision of immobilization on a polymer carrier of an unstable water-soluble BAS, that is, a BAS that is destroyed when immobilized by the other methods, Specifically, under the conditions created by the closest-art method where ionizing radiation is used in doses of 0.5 to 1 Mrad and polymer concentrations are 5 to 10%; in cases where a BAS is not destroyed completely during immobilization, the technical result consists of an increased percent survival of the BAS.

This technical result under the conditions of the method of the present invention is provided by freezing the reaction mixture at temperature ranging from -20°C to -140°C before irradiating it.

As stated above, both variations of the method are intended for immobilizing water-soluble BASs. In cases where a particular BAS is poorly soluble in a neutral aqueous medium, solution pH is brought to the required value by means of a pharmacologically acceptable chemical, for example, acetic acid.

A BAS-carrier conjugate obtained by the above-described methods is characterized in that the BAS is selected from the group comprising proinsulin, tyrosyl-2-alanyl-glycyl-phenylalanyl-leucyl-arginine diacetate, alcalase, hyaluronidase, and somatotropin and in that the carrier is a pharmacologically acceptable water-soluble polymer which is selected, for example, from the group comprising polyethylene oxide, polyvinylpyrrolidone, dextran, Pluronic, and acrylamide, wherein a BAS molecule is covalently linked to at least one polymer molecule.

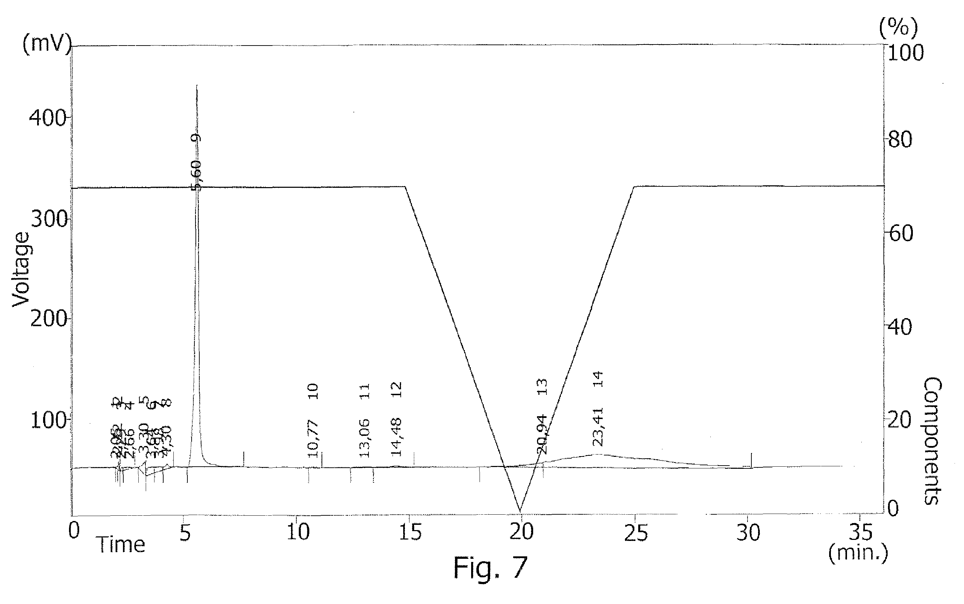

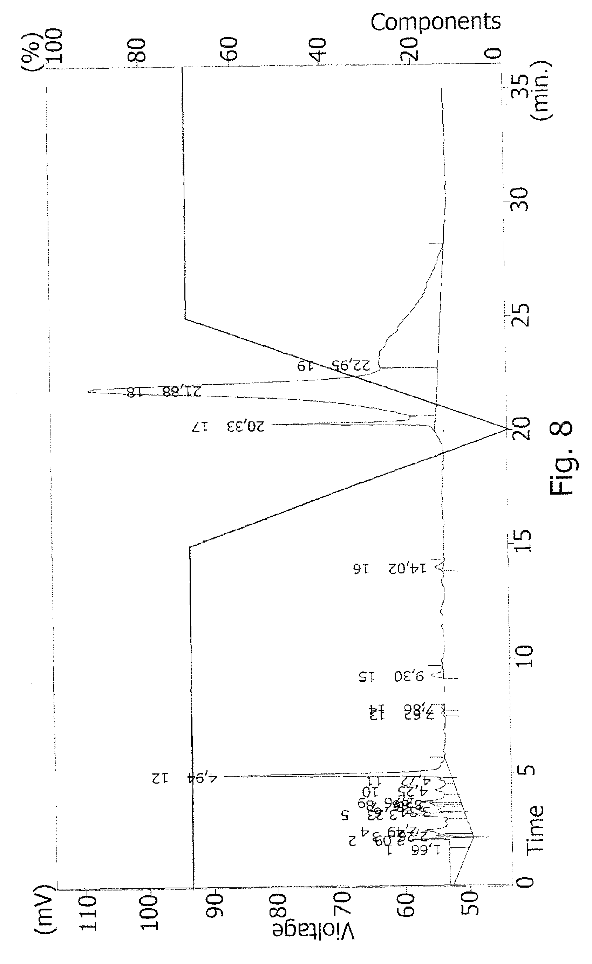

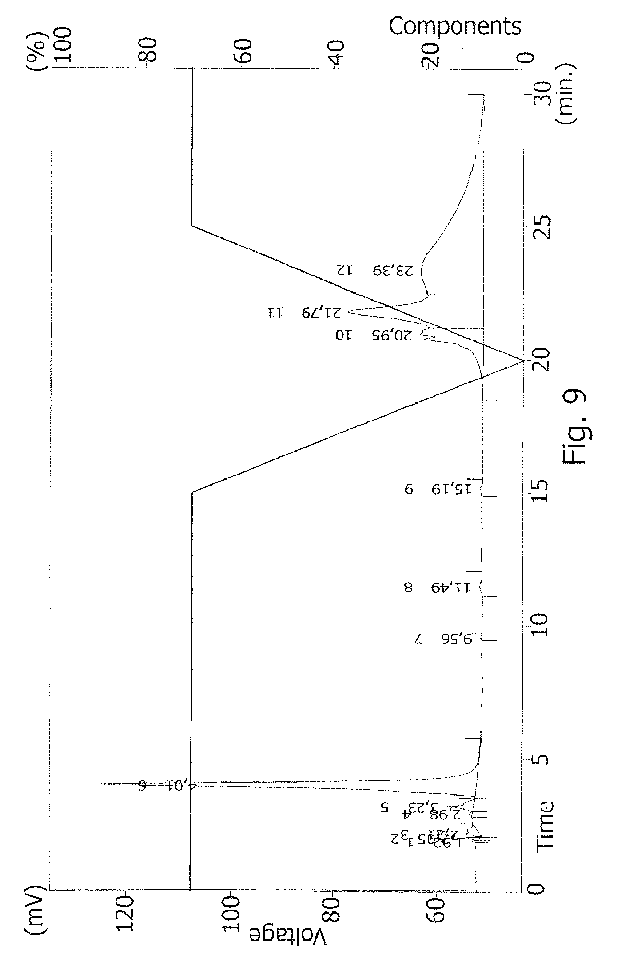

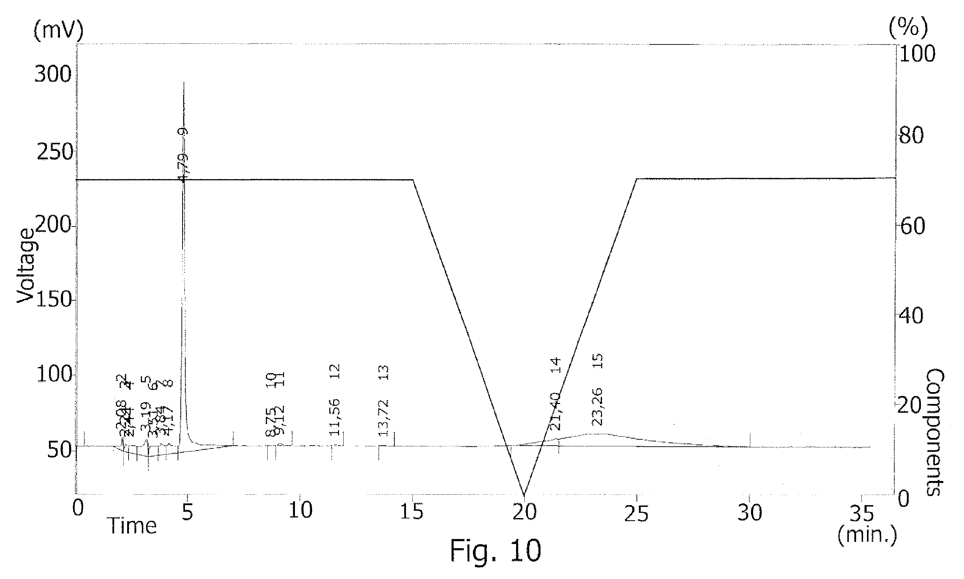

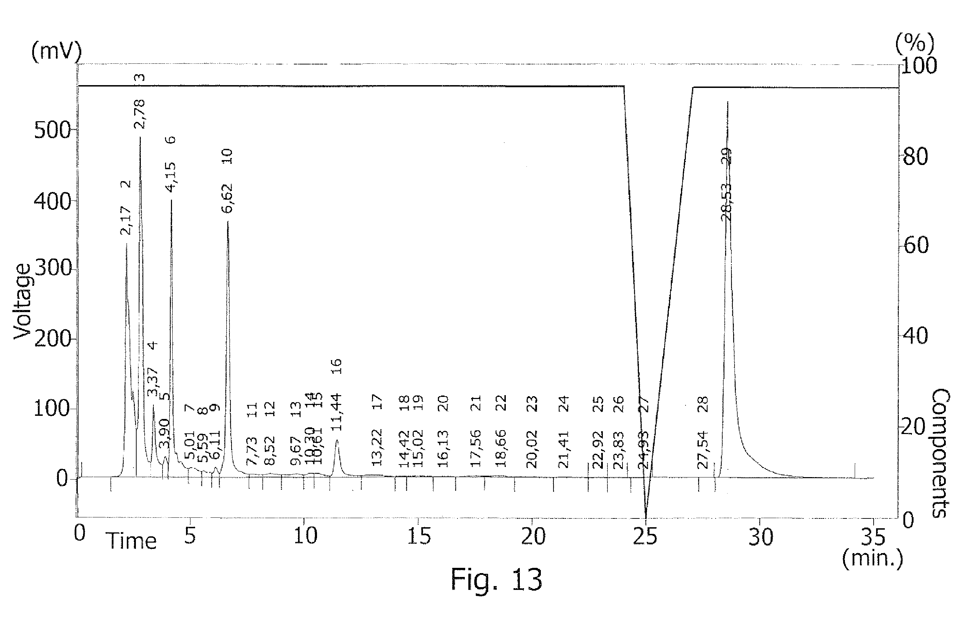

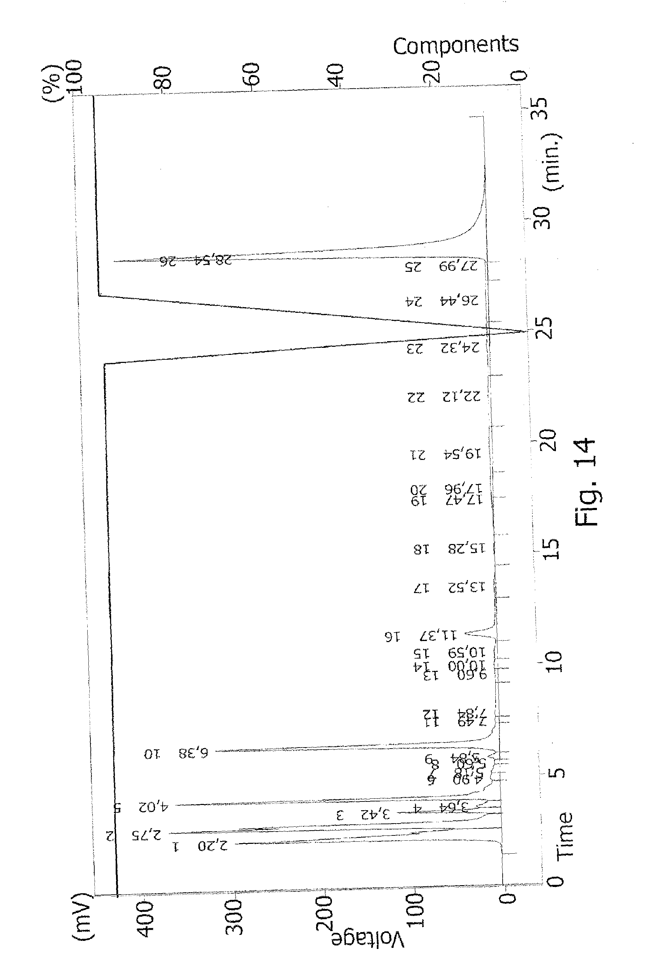

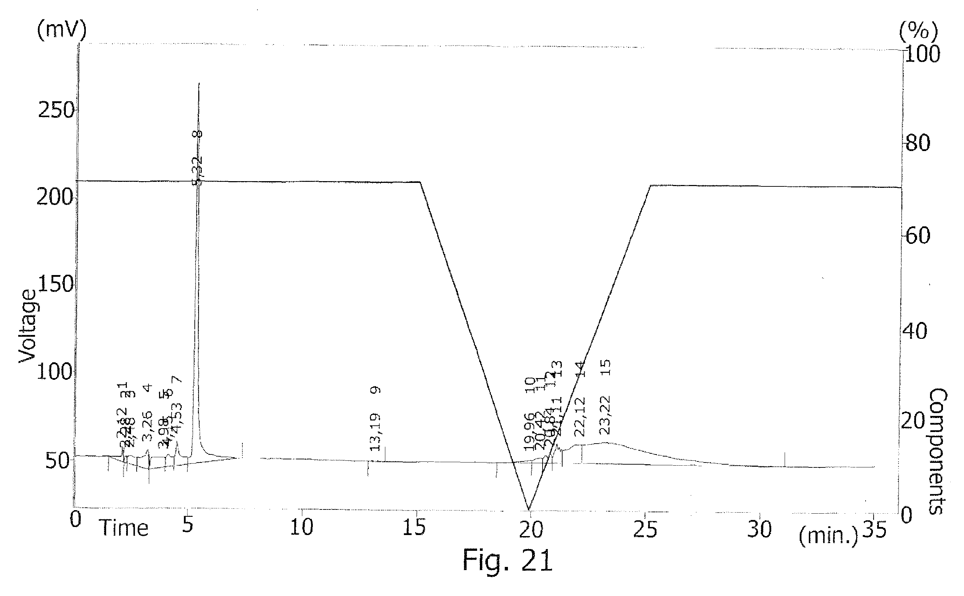

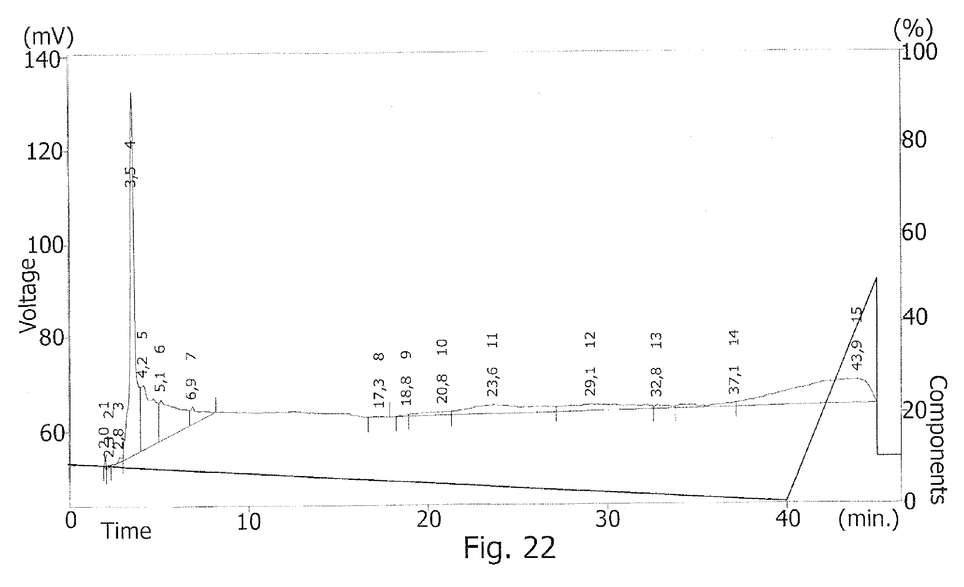

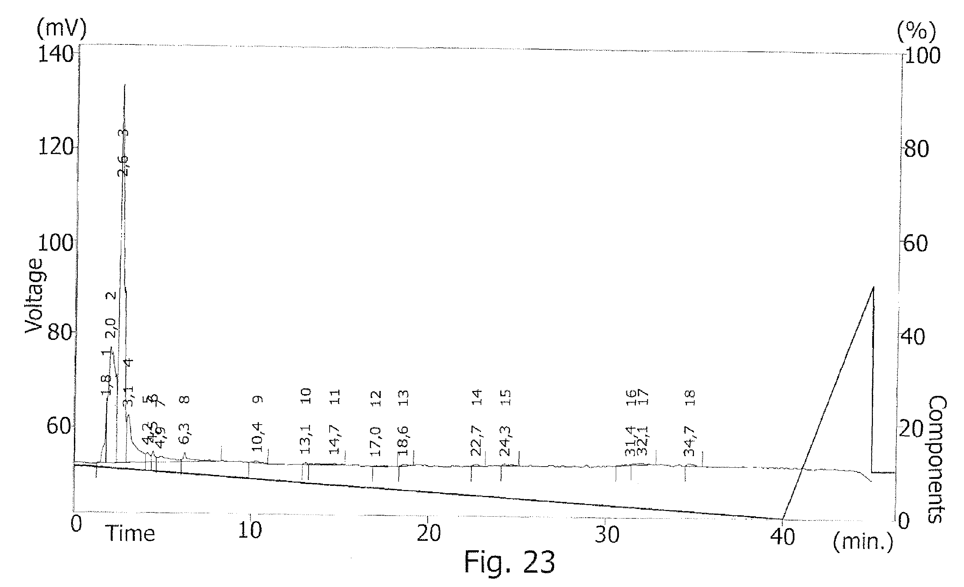

Some specific conjugates are as follows:
- tyrosyl-2-alanyl-glycyl-phenylalanyl-leucyl-arginine diacetate - polyvinylpyrrolidone (Plasdone K-17) conjugate (see Example 4, Figs. 7, 8, 9; Example 5, Figs. 10, 9; Example 12, Figs. 21, 8);
- alcalase - polyethylene oxide-300 conjugate (polyethylene oxide having a molecular mass of 300 Da) (see Example 2; Table);
- hyaluronidase - dextran 70 conjugate (dextran having a molecular mass of 70 kDa) (see Example 8, Figs. 13, 14);
- proinsulin - Pluronic F-68 conjugate (see Example 9, Figs. 15, 16; Example 10, Figs. 17, 18);
- somatotropin - dextran conjugate (dextran having a molecular mass of 40 to 60 kDa) (see Example 14, Figs. 22, 23).

### Brief Description of the Drawings

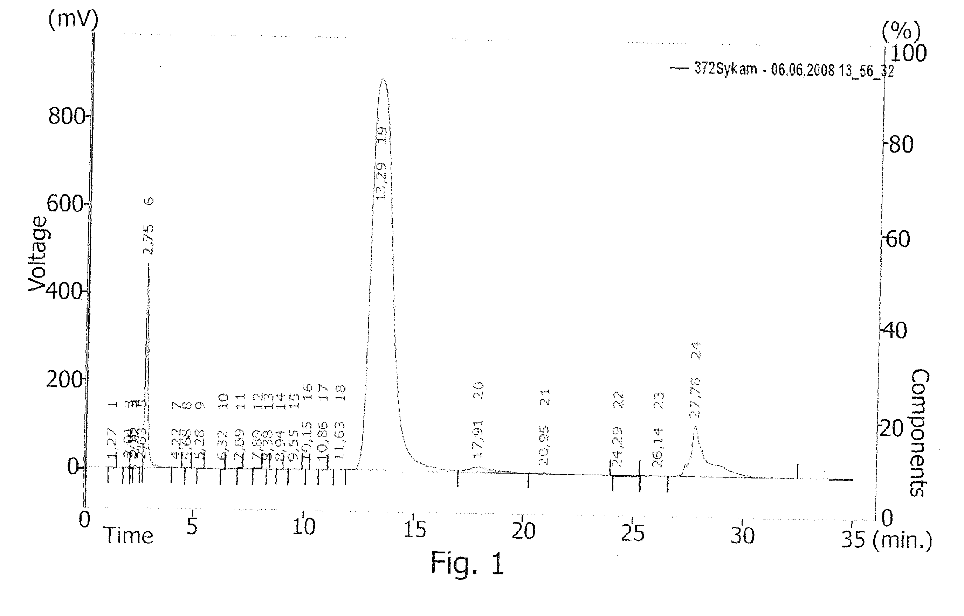

Fig. 1 shows an HPLC profile for an insulin solution in 10% polyethylene oxide-1500.

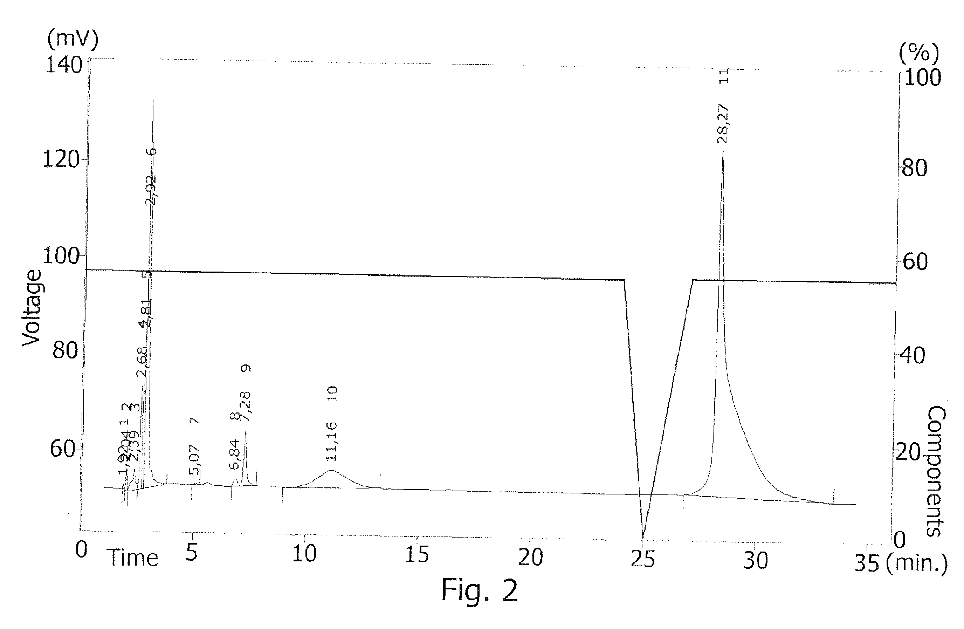

Fig. 2 shows an HPLC profile for an insulin solution in 10% polyethylene oxide-1500 after it was irradiated with ionizing radiation in a dose of 0.5 Mrad.

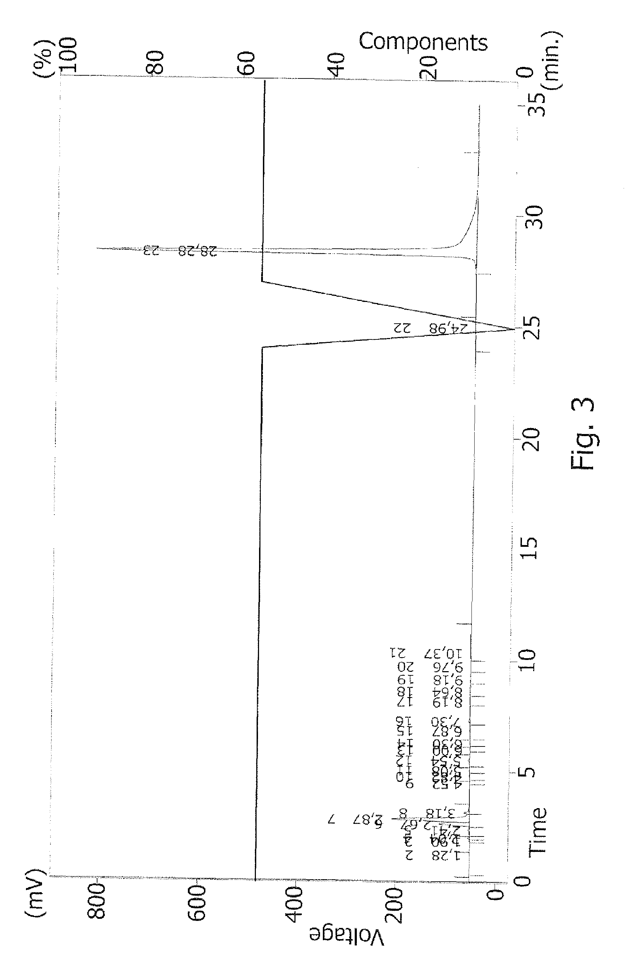

Fig. 3 shows an HPLC profile for an insulin solution in 10% polyethylene oxide-1500 after it was irradiated with ionizing radiation in a dose of 1.5 Mrad.

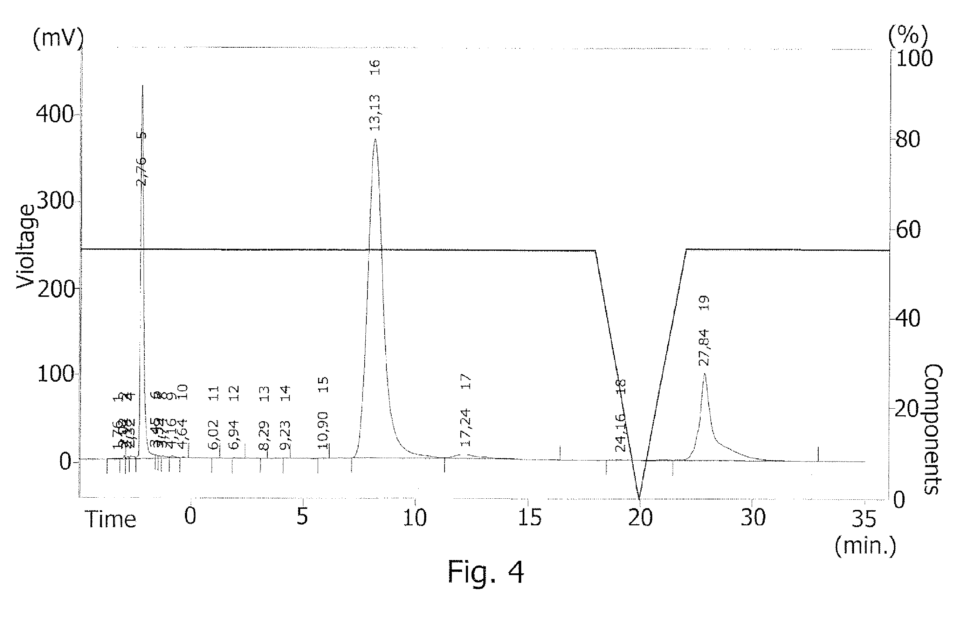

Fig. 4 shows an HPLC profile for an insulin solution in 20% polyethylene oxide-1500.

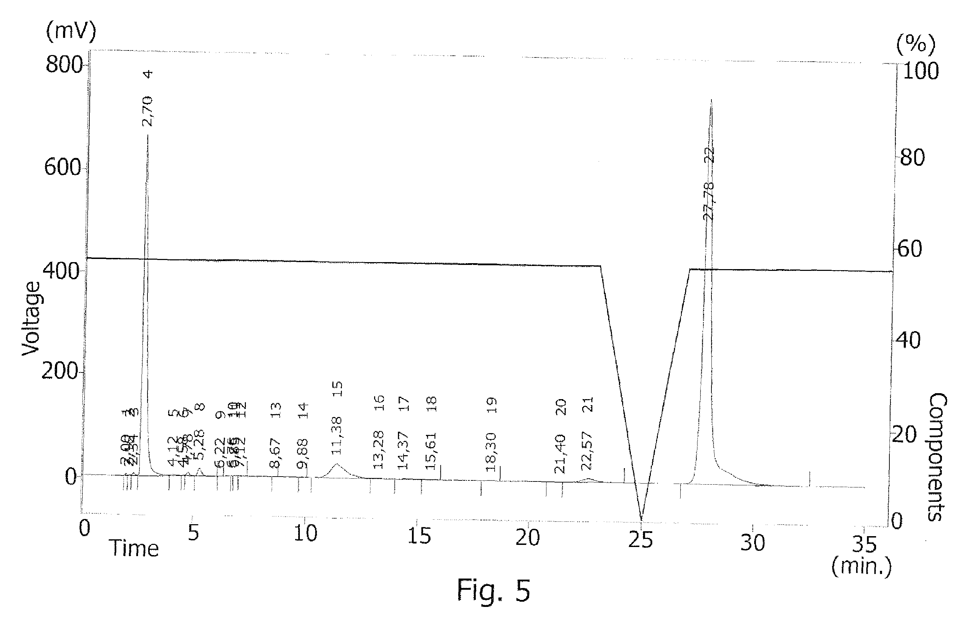

Fig. 5 shows an HPLC profile for an insulin solution in 20% polyethylene oxide-1500 solution after it was irradiated with an accelerated electron beam in a dose of 1.0 Mrad.

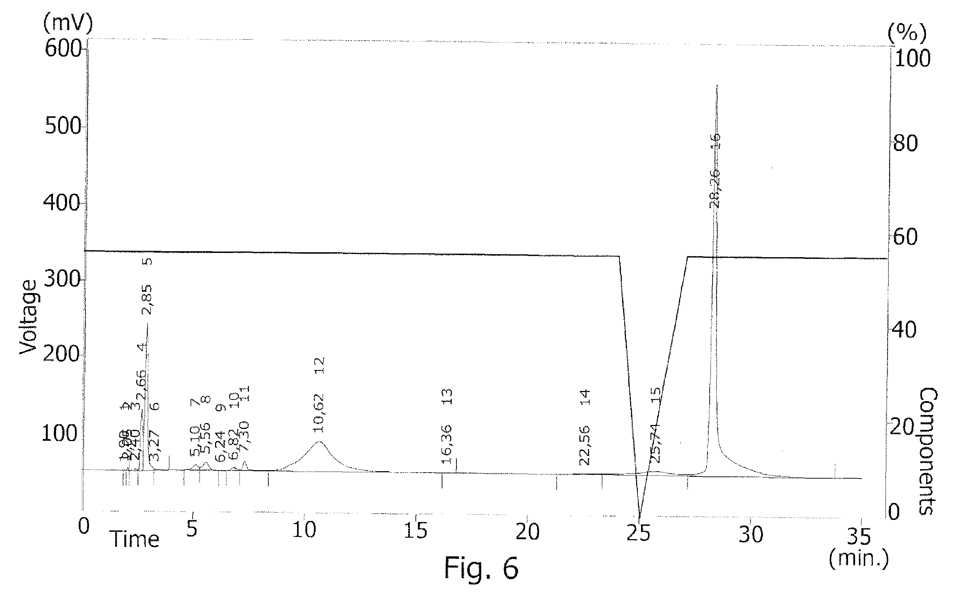

Fig. 6 shows an HPLC profile for an insulin solution in 75% polyethylene oxide-1500 after it was irradiated with an accelerated electron beam in a dose of 1.0 Mrad,
Fig. 7 shows an HPLC profile for an aqueous solution of tyrosyl-2-alanyl-glycyl-phenylalanyl-leucyl-arginine diacetate.
Fig. 8 shows an HPLC profile for a solution of tyrosyl-2-alanyl-glycyl-phenylalanyl-leucyl-arginine diacetate in 11% Plasdone K-17 solution after it was irradiated with an accelerated electron beam in a dose of 1.0 Mrad.
Fig. 9 shows an HPLC profile for a solution of tyrosyl-2-alanyl-glycyl-phenylalanyl-leucyl-arginine diacetate in a 50% Plasdone K-17 solution after it was irradiated with an accelerated electron beam in a dose of 1.0 Mrad.
Fig. 10 shows an HPLC profile for a solution of tyrosyl-2-alanyl-glycyl-phenylalanyl-leucyl-arginine diacetate in 50% Plasdone K-17 solution after it was frozen to -20°C and then irradiated with an accelerated electron beam in a dose of 1.0 Mrad.

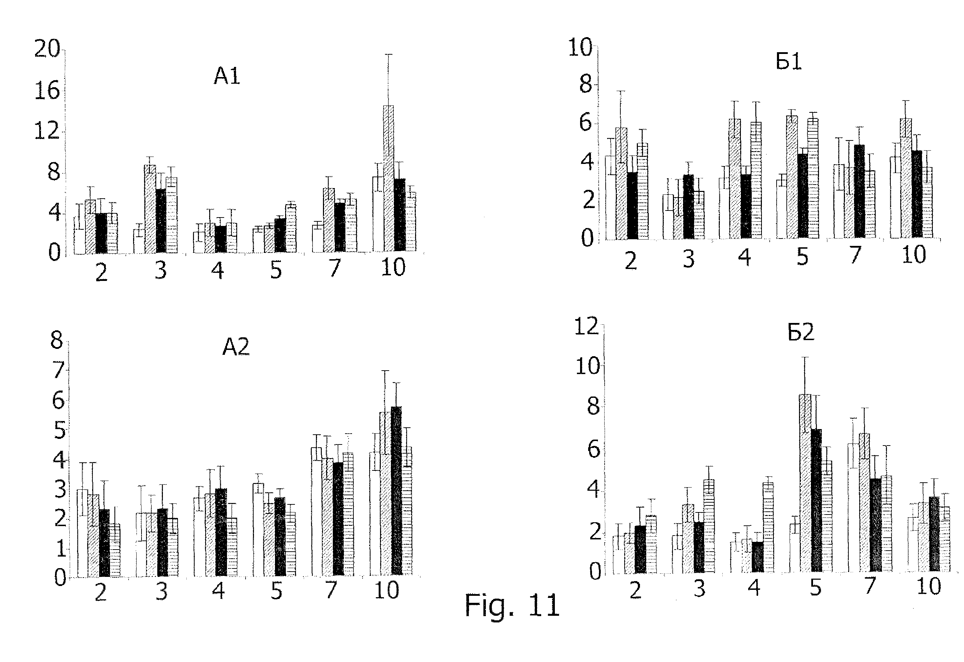

Fig. 11 shows CFU-GM (A) and CFU-E (B) counts in bone marrow (index-1) and in peripheral blood (index-2) in CBA/Calac line mice upon administration of a physiological saline (white bars), subcutaneous administration of immobilized gCSF (oblique-hatched bars), parenteral administration of immobilized gCSF (black bars), and subcutaneous administration of unconjugated GCSF, (horizontal-hatched bars). Sample times (days) are plotted along the abscissa; index values are plotted along the ordinate: A for 10⁵ myelokaryocytes; B for 10⁵ mononuclears. Confidential ranges are given for p<0.05.

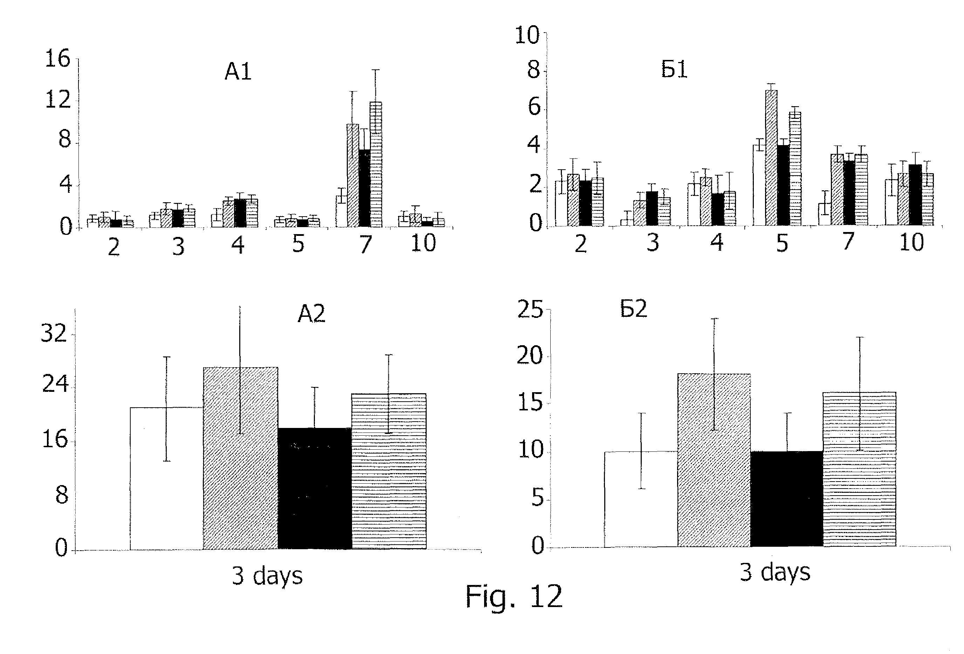

Fig. 12 shows CFU-F (A) and MSC (B) counts in bone marrow (index-1) and in peripheral blood (index-2) in CBA/Calac line mice upon administration of a physiological saline (white bars), subcutaneous administration of immobilized gCSF (oblique-hatched bars), parenteral administration of immobilized gCSF (black bars), and subcutaneous administration of unconjugated gCSF (horizontal-hatched bars). Sample times (days) are plotted along the abscissa; index values are plotted along the ordinate: A1 for 2.5 × 10⁵ myelokaryocytes; A2 for 2.5 × 10⁵ mononuclears; B1 for 10⁶ myelokaryocytes; and B2 for 2.5 × 10⁶ mononuclears. Confidential ranges are given for p<0.05.
Fig. 13 shows HPLC data for a hyaluronidase solution in a 25% dextran-70 solution (dextran having a molecular mass of 70 kDa).
Fig. 14 shows HPLC data for a hyaluronidase solution in a 25% dextran-70 solution after it was irradiated with bremsstrahlung γ-rays in a dose of 0.5 Mrad.

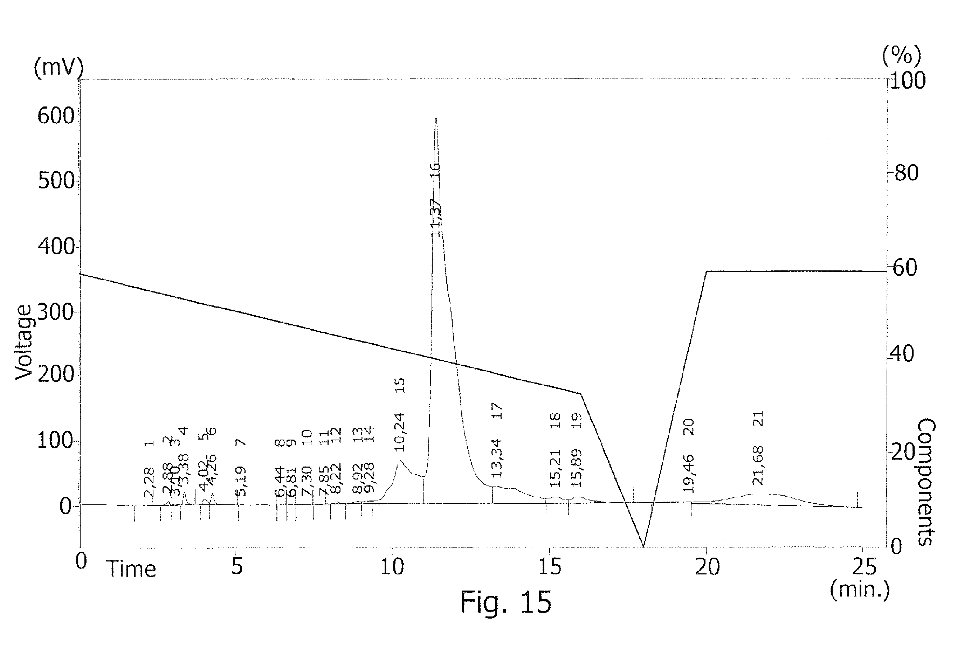

Fig. 15 shows HPLC data for a proinsulin solution in a 20% Pluronic F-68 solution.

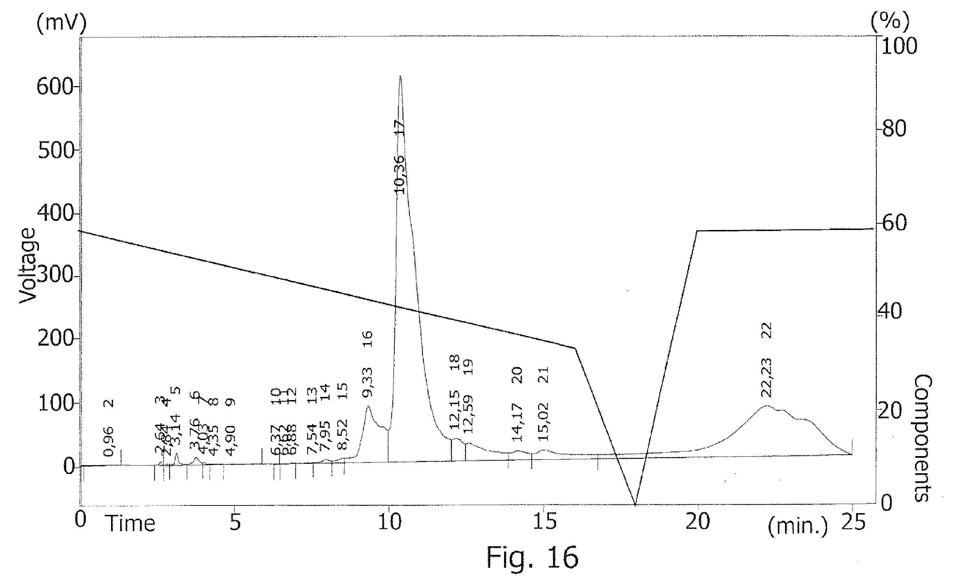

Fig. 16 shows HPLC data for a proinsulin solution in a 20% Pluronic F-68 solution after it was frozen to -70°C and then irradiated with an accelerated electron beam in a dose of 1.0-Mrad.

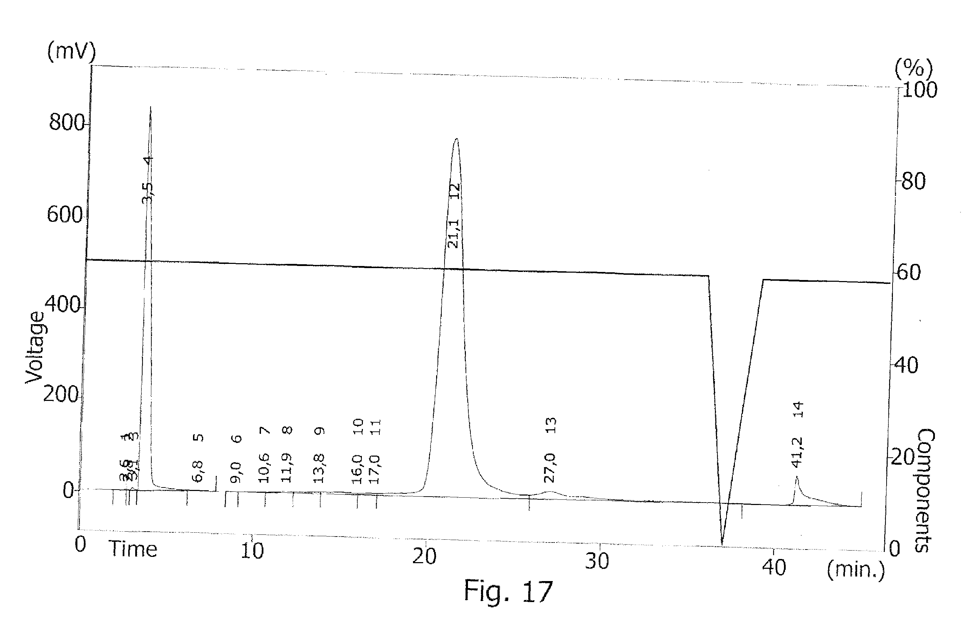

Fig. 17 shows HPLC data for an insulin solution in a 20% Pluronic F-68 solution.

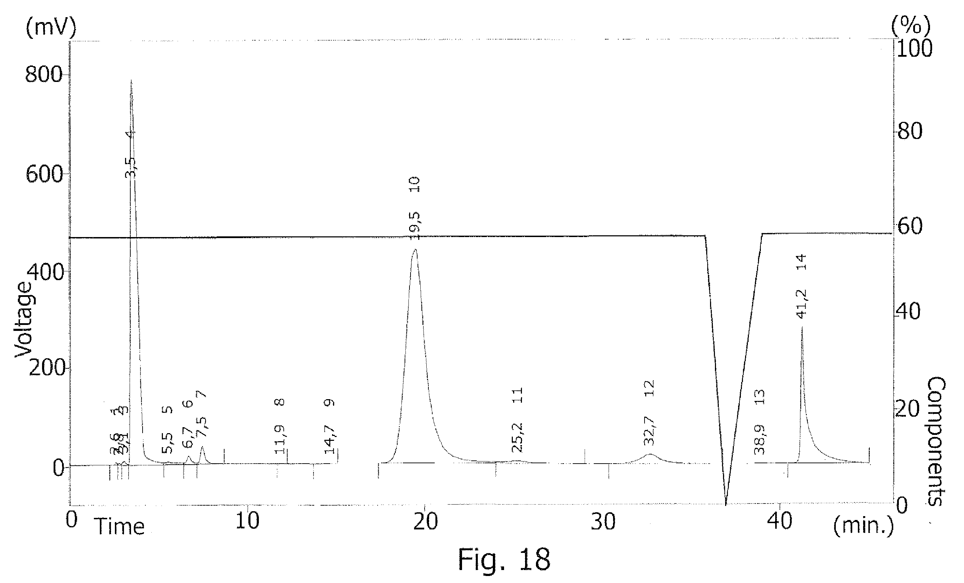

Fig. 18 shows HPLC data for an insulin solution in a 20% Pluronic F-68 solution after it was frozen to -20°C and then irradiated with an accelerated electron beam in a dose of 1.5 Mrad.

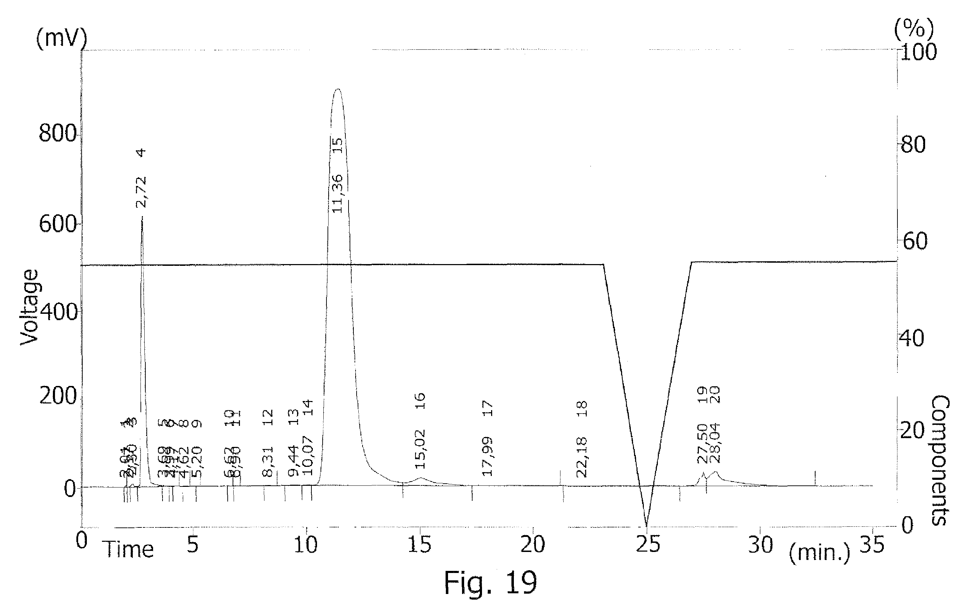

Fig. 19 shows HPLC data for an insulin solution in a 20% polyethylene oxide-1500 solution.

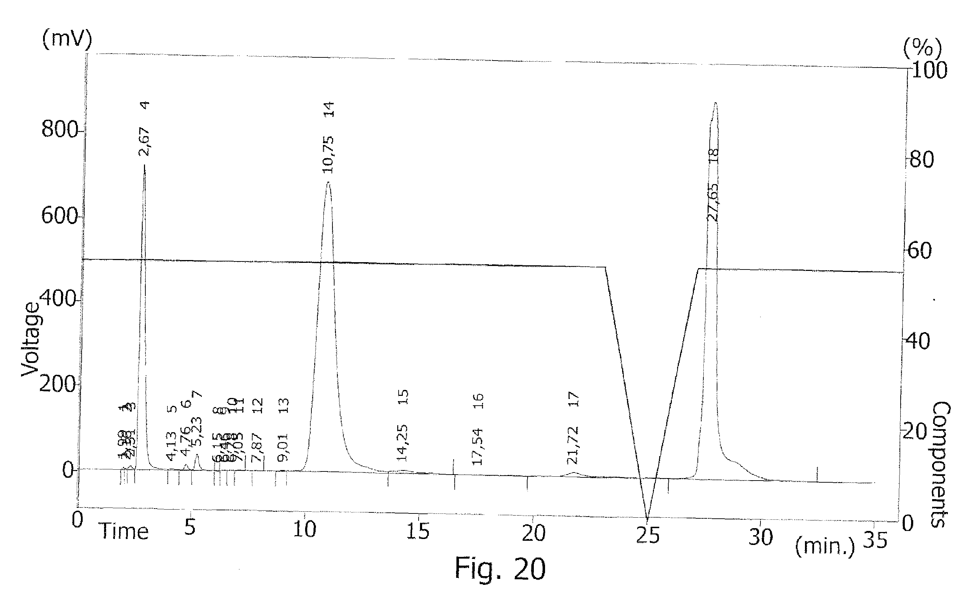

Fig. 20 shows HPLC data for an insulin solution in a 20% polyethylene oxide-1500 solution after it was frozen to -140°C and then irradiated with an accelerated electron beam in a dose of 5 Mrad.
Fig. 21 shows an HPLC profile for a tyrosyl-2-alanyl-glycyl-phenylalanyl-leucyl-arginine diacetate solution in a 10% Plasdone K-17 solution after it was frozen to -70°C and then irradiated with an accelerated electron beam in a dose of 1.0 Mrad.
Fig. 22 shows HPLC data for a somatotropin solution in 10% dextran.
Fig. 23 shows HPLC data for a somatotropin solution in 10% dextran after it was frozen to -20°C and then irradiated with bremsstrahlung γ-rays in doses of 0.5 Mrad.

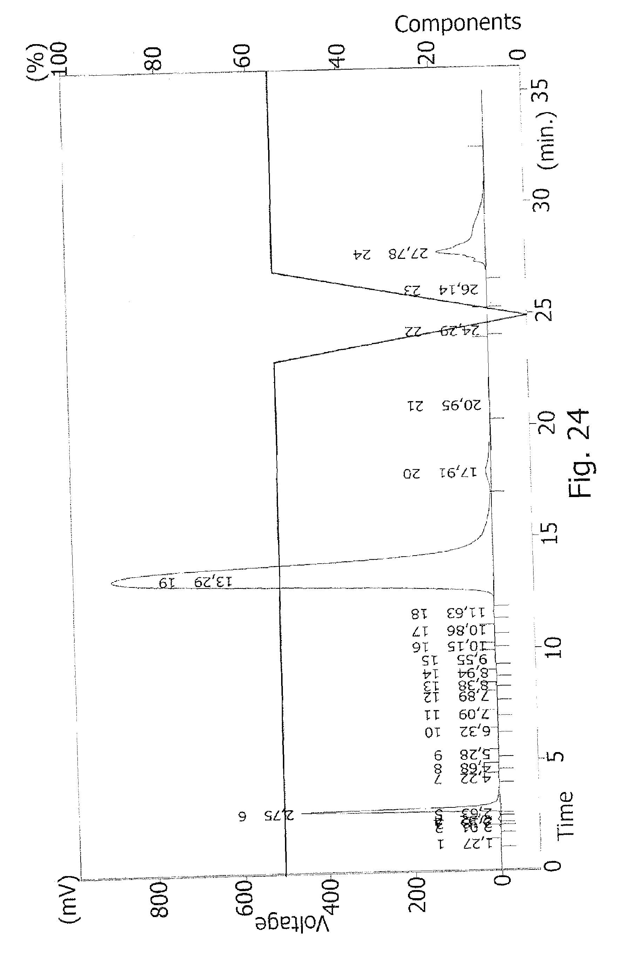

Fig. 24 shows HPLC data for an insulin solution in 10% polyethylene oxide-1500 solution.

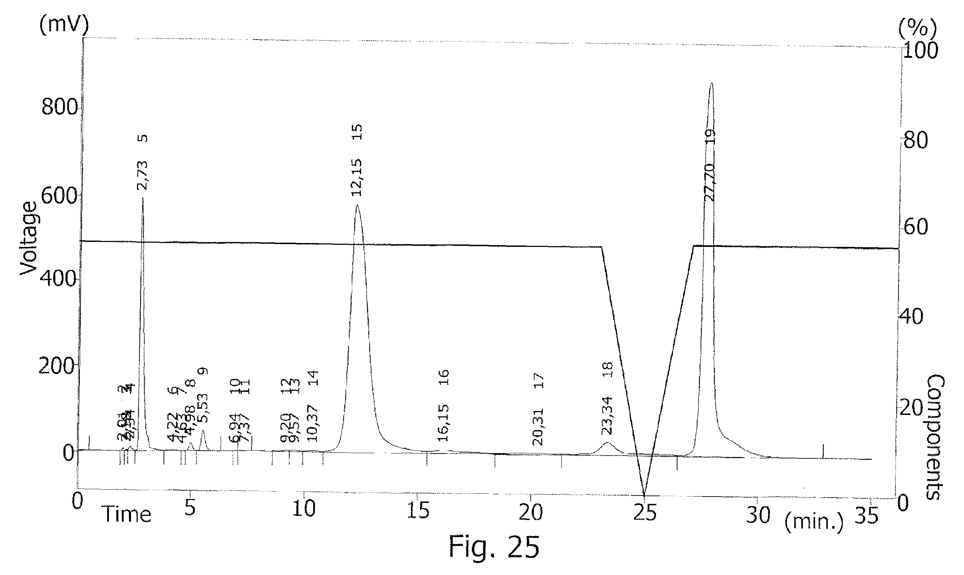

Fig. 25 shows HPLC data for an insulin solution in a 10% polyethylene oxide-1500 solution after it was frozen to -20°C and then irradiated with an accelerated electron beam in a dose of 1 Mrad.

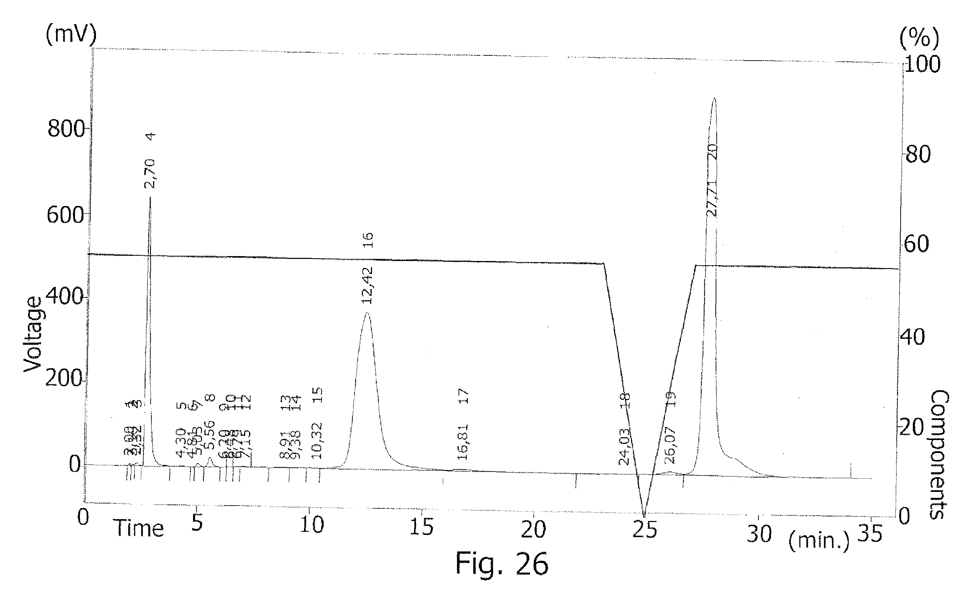

Fig. 26 shows HPLC data for an insulin solution in a 10% polyethylene oxide-1500 solution after it was frozen to -70°C and then irradiated with an accelerated electron beam in a dose of 1.5 Mrad.

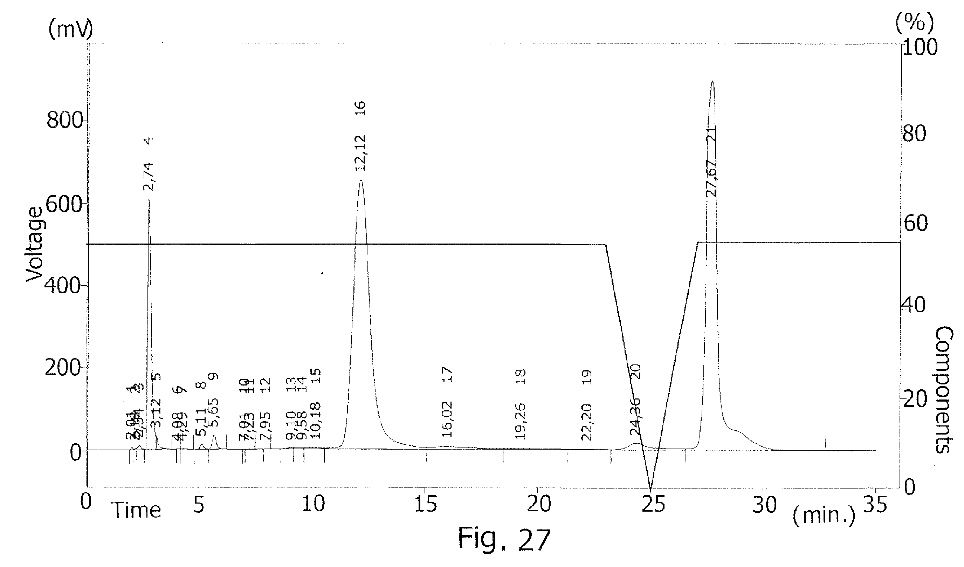

Fig. 27 shows HPLC data for an insulin solution in a 10% polyethylene oxide-1500 solution after it was frozen to -140°C and then irradiated with an accelerated electron beam in a dose of 5 Mrad.

In the figures showing HPLC profiles, the abscissa plots time in minutes and the ordinate plots voltage in millivolts. The time is plotted along axis X on the same scale; the voltage is plotted along axis Y on various scales. The figures at the peak points of peaks have the following meaning: the lower figure indicates time in minutes at the peak (corresponding to the maximal yield of the substance referring to this peak), and the upper figure indicates the peak number.

Further, examples are provided to illustrate the invention described herein; that is, these examples are intended for the better understanding of the present invention, and do not limit in any way the scope of the claims which follow.

### Example 1. An unstable BAS (insulin) is destroyed completely or almost completely when immobilized by the closest-art method.

In 5 ml of a 10% polyethylene oxide-1500 solution, dissolved was a 10-ml portion of recombinant human insulin. A sample was taken for analysis by high-performance liquid chromatography (hereinafter, HPLC).

Next, the solution was transferred to two polyethylene bags, both sealed air tight, and then irradiated with ionizing radiation. The exposure dose was 0.5 Mrad for the first bag and 1.5 Mrad for the second one. The solutions from the irradiated bags were again sampled for carrying out HPLC. Reversed-phase HPLC was carried out on a Sykam chromatograph system (Germany) using a Phenomenex Luna C18 5-µm 100A column (USA). The isocratic mode was employed: phase A, 55%; phase B, 45%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 214 nm.

The results of HPLC are displayed in Fig. 1 (for the intact solution), Fig. 2 (for the solution irradiated with a dose of 0.5 Mrad), and Fig. 3 (for the solution irradiated with a dose of 1.5 Mrad). Peak 18 in Fig. 1 (time: 10.77 min) and peak 10 in Fig. 2 (time: 11.16 min) are insulin peaks. In Fig. 3, there is no insulin peak.

The insulin peak area ratio was used to calculate that 2.3% of the initial insulin amount survived in the solution after exposure to a radiation dose of 0.5 Mrad (Fig. 2). When exposed to 1.5 Mrad, insulin was destroyed completely (Fig. 3).

Thus, an unstable BAS (in the case at hand, insulin) is destroyed almost completely when immobilized under the conditions of the closest-art process.

### Example 2

Two samples were prepared. In sample No. 1, 5 ml of concentrated alcalase (from Novozymes A/S) was dissolved in 45 ml of a 50% polyethylene oxide-300 solution (polyethylene oxide having a molecular mass of 300 Da); in sample No. 2, 5 ml of concentrated alcalase was dissolved in 45 ml of a 20% polyethylene oxide-300 solution. The blank was a solution of 5 ml of concentrated alcalase in 45 ml of water. The initial proteolytic activity of alcalase in the above solutions was measured by means of hydrolysis of 1% sodium caseinate solution.

The reaction mixtures were sealed in polyethylene bags and then irradiated on an ILU-6 electron accelerator with a dose of 1.5 Mrad in order to immobilize alcalase on polyethylene oxide-300.

After irradiation, the proteolytic activity of alcalase in the samples was measured again. The results of these measurements are displayed in the table below.

Alcalase proteolytic activity measurement data (U/ml)

| Alcalase activity | Blank | Sample No. 1 | Sample No. 2 |
|---|---|---|---|
| Intact | 2381 | 2389 | 2368 |
| Immobilized | | 2338 | 1446 |

The data displayed in the table show that the activity of immobilized alcalase in the sample where the polyethylene oxide-300 percentage was 20% is considerably lower than in the sample where the polyethylene oxide percentage was 50%.

Thus, the polymer concentration has a considerable effect on the survival of an unstable BAS; at a higher polymer concentration, a greater percentage of alcalase survives.

### Example 3

Two samples were prepared. In sample No. 1, a 10-mg portion of recombinant human insulin was dissolved in 5 ml of a 20% polyethylene oxide-1500 solution. In sample No. 2, a 10-mg portion of recombinant human insulin was dissolved in 5 ml of a 75% polyethylene oxide-1500 solution.

From sample No. 1, a sample was taken to be analyzed by HPLC.

The solutions were transferred to polyethylene bags, sealed air-tight, and then irradiated on an ILU-6 electron accelerator with a dose of 1.0 Mrad.

From both bags, solutions were sampled for carrying out repeated HPLC. Reversed-phase HPLC was carried out on a Sykam chromatograph system (Germany) using a Phenomenex Luna C18 5-µm 100A column (USA). The isocratic mode was employed: phase A, 55%; phase B, 45%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 214 nm.

The results of HPLC for a nonirradiated insulin solution in 20% polyethylene oxide-1500 solution are shown in Fig. 4. The insulin peak is peak 16.

The results of HPLC for irradiated solutions are shown in Fig. 5 (for sample No. 1) and Fig. 6 (for sample No. 2). The insulin peaks are peak 15 in Fig. 5 and peak 12 in Fig. 6.

In a 20% polyethylene oxide solution (sample No. 2) after it was irradiated with a dose of 1.0 Mrad, 9% of the initial insulin amount survived; in a 75% polyethylene oxide solution (sample No. 3), the percent survival was about 25%. In this way, an increase in polymer concentration in solution results in a greater percent survival of immobilized protein molecules under an impact of ionizing radiation. The shift of the insulin peak signifies that the BAS has been immobilized on the polymer and that an insulin - polyethylene oxide-1500 conjugate has been formed.

### Example 4

Three samples were prepared. In sample No. 1, 2 mg of tyrosyl-2-alanyl-glycyl-phenylalanyl-leucyl-arginine diacetate was dissolved in 1 ml of water (a blank).

In sample No. 2, 2 mg of tyrosyl-2-alanyl-glycyl-phenylalanyl-leucyl-arginine diacetate was mixed with 1 ml of a 11% Plasdone K-17 solution (Plasdone K-17 is polyvinylpyrrolidone having MM of 17 000 Da); in sample No. 3, 2 mg of tyrosyl-2-alanyl-glycyl-phenylalanyl-leucyl-arginine diacetate was mixed with 1 ml of a 50% Plasdone K-17 solution.

The solutions were sealed in air-tight polyethylene bags; the samples were irradiated on an ILU-6 electron accelerator with a dose of 1.0 Mrad. Following this, reversed-phase HPLC was carried out on a Sykam chromatograph system (Germany) using a Phenomenex Luna C18 5-µm 100A column (USA). The isocratic mode was employed: phase A, 55%; phase B, 45%, wherein phase A is 0.1 M Na₂SO₄ solution (pH 2.3) (90%) + acetonitrile (10%) and phase B is 0.1 Na₂SO₄ solution (pH 2.3) (50%) + acetonitrile (50%); detection was at 230 nm.

The results of HPLC are displayed in Fig. 7 (blank), Fig. 8 (sample No. 2), and Fig. 9 (sample No. 3). The tyrosyl-2-alanyl-glycyl-phenylalanyl-leucyl-arginine diacetate peaks are peak 9 in Fig. 7, peak 12 in Fig. 8, and peak 6 in Fig. 9.

In sample No. 2, about 10% of the initial tyrosyl-2-alanyl-glycyl-phenylalanyl-leucyl-arginine diacetate amount survived; in sample No. 3, the percent survival was about 30%. An increase in polymer concentration in solution resulted in a greater percent survival of the BAS. The shift of the tyrosyl-2-alanyl-glycyl-phenylalanyl-leucyl-arginine diacetate peak signifies that the BAS has been immobilized on the polymer and that a tyrosyl-2-alanyl-glycyl-phenylalanyl-leucyl-arginine diacetate - polyvinylpyrrolidone (Plasdone K-17) conjugate has been formed.

### Example 5

A sample was prepared as follows: 2 mg of tyrosyl-2-alanyl-glycyl-phenylalanyl-leucyl-arginine diacetate was mixed with 1 ml of a 50% Plasdone K-17 solution. The solution was sealed in an air-tight polyethylene bag.

The sample contained in the polyethylene bag was placed into a freezer maintained at - 70°C and stored there for 16 hours, followed by irradiation on an ILU-6 electron accelerator with a dose of 1.0 Mrad. Reversed-phase HPLC was carried out on a Sykam chromatograph system (Germany) using a Phenomenex Luna C18 5-µm 100A column (USA). The isocratic mode was employed: phase A, 55%; phase B, 45%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 230 nm. The results of HPLC are displayed in Fig. 10. The tyrosyl-2-alanyl-glycyl-phenylalanyl-leucyl-arginine diacetate peak is peak 9.

The tyrosyl-2-alanyl-glycyl-phenylalanyl-leucyl-arginine diacetate peak area ratio was calculated to find out that the percent survival of immobilized tyrosyl-2-alanyl-glycyl-phenylalanyl-leucyl-arginine diacetate in the frozen sample was almost two times higher than for a similar sample exposed to ionizing radiation in a liquid form (Example 4, Fig. 9). The shift of the tyrosyl-2-alanyl-glycyl-phenylalanyl-leucyl-arginine diacetate peak signifies that the BAS has been immobilized on the polymer and that a tyrosyl-2-alanyl-glycyl-phenylalanyl-leucyl-arginine diacetate - polyvinylpyrrolidone (Plasdone K-17) conjugate has been formed.

It follows that freezing of a solution before irradiating it improves the survival of a polymer-immobilized BAS.

### Example 6

To 5 ml of a 20% polyethylene oxide-1500 solution, added was a 50-mg portion of recombinant human insulin (from OAO National Biotechnologies, town of Obolensk, Russia). Inasmuch as this grade of insulin is soluble in acid media and insoluble in neutral media such as polyethylene oxide solutions, glacial acetic acid was added to the mixture to bring pH to a value between 2.5 and 2.8 as monitored with a pH meter. As a result of acid hydrolysis, insulin dissolves in polyethylene oxide-1500, and insulin + polyethylene oxide mixtures become clear. If a sample of a solution prepared in this way is taken and its pH is shifted toward 7.0, insulin will precipitate.

The solution was transferred to a polyethylene bag and sealed air tight. The bag was placed to a freezer maintained at -70°C and kept there for 12 hours. Following this, the bag was irradiated on an ILU-6 electron accelerator with a dose of 1.0 Mrad.

After irradiation, insulin did not precipitate when the solution was alkalinized to pH of 9. In this way, evidence was obtained that covalent linkage appeared between the BAS and carrier polymer and that an insulin - polyethylene oxide-1500 conjugate has been formed.

### Example 7

gCSF immobilization on polyethylene oxide-1500 was performed as follows: 2.5 ml of the gCSF substance produced by OAO National Biotechnologies (0.4 mg/ml in acetate buffer) was mixed with 2.5 ml of 20% polyethylene oxide-1500. The mixture was poured to a polyethylene bag and frozen at -70°C for 16 hours. Following this, the bag was irradiated on an ILU-6 electron accelerator with a dose of 1.5 Mrad to produce immobilized gCSF.

Experiments were performed on 250 CBA/Calac line mice aged 2 months. Nonimmobilized gCSF was administered subcutaneously in doses of 140 µg/kg for 5 days. Immobilized gCSF (a gCSF - polyethylene oxide-1500 conjugate) was administered also in doses of 100 µg/kg (based on the active agent) subcutaneously for 5 days and per os for 10 days. Comparison mice received a physiological saline in an equivalent volume (0.2 ml) in the same schedules.

On the 2nd, 3rd, 4th, 5th, 7th, and 10th days of the experiment, the bone marrow and peripheral blood counts of granulomonocytic (GM), erythroid (E), and fibroblast (F) colony forming units (CFU) were determined in the experimental and comparison sets of animals by cloning in a semiviscous culture medium. Mesenchymal stem cells (MSC) counts in bone marrow and peripheral blood were studied on the 3rd day of the experiment using limiting dilutions.

Administration of the medications revealed a considerable effect thereof on the condition of the parent cell pool. gCSF, which is the active agent of all the medications studied, in all cases provided an increase in the counts of granulomonocytic precursors in in the hematopoietic tissue. For example, in using unconjugated gCSF and in intragastrically administering immobilized gCSF, CFU-GM counts increased on the 3rd, 5th, and 7th days of the experiment (Fig. 11, A1). However, subcutaneous administration of immobilized gCSF gave a longer (on 3rd, 7th, and 10th days) and most pronounced (on the 3rd day of the study, up to 372.1% against the relevant value in comparison mice) increase in hematopoietic cell counts in bone marrow (Fig. 11).

The subcutaneous administration of immobilized gCSF leads on the 5th day to a significant increase in peripheral blood CFU-E counts against unconjugated gCSF (Fig. 11, B2).

The results of the experiments prove that the biological activity is retained, bioavailability is enhanced (oral administration is possible), and the exposure time of gCSF is increased.

Similar alterations were observed in the pool of fibroblast colony forming units containing both committed stromal elements and multipotent stem cells. The administration of unconjugated and immobilized gCSF medications (in both schedules) increased CFU-F counts in the hematopoietic tissue on the 3rd, 4th, 7th and the 4th, 7th days of the experiment, respectively. Ingestion of the medication immobilized using nanotechnologies had a less pronounced effect (on the 7th day) compared to the parenteral administration of conventional gCSF. The aforementioned alterations of the CFU-F functional activity in bone marrow were accompanied by an enhancement of yield thereof to peripheral blood. Importantly, this response was most prominent in the set of animals that received immobilized gCSF subcutaneously and less prominent in the mice that received immobilized cytokine intragastrically (Fig. 12).

The subcutaneous administration of conjugated gCSF on the 5th day gave a significant increase in bone marrow MSC counts against unconjugated gCSF (Fig. 12, B2).

The figures make it clear that gCSF immobilized by the method of the present invention has a prominent biological effect exceeding the effect of nonimmobilized gCSF.

### Example 8

In 3 ml of a 25% dextran 70 solution (where dextran had a molecular mass of 70 kDa), dissolved was a 100-mg portion of hyaluronidase. A sample for HPLC was taken. Next, the solution was transferred to a polyethylene bag, sealed air tight, and frozen at -20°C for 16 hours. Following this, the bag was exposed to bremsstrahlung γ-rays in a dose of 0.5 Mrad. From the / irradiated bag, a sample was again taken for HPLC. Reversed-phase HPLC was carried out on a Sykam chromatograph system (Germany) using a Phenomenex Luna C18 5-µm 100A column (USA). The isocratic regime was employed: phase A, 95%; phase B, 5%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 214 nm.

The results of HPLC are displayed in Fig. 13 and Fig. 14. The hyaluronidase peak is peak 10 in Figs. 13 and 14.

After exposure to ionizing radiation, about 85% of the hyaluronidase survived. The shift of the HPLC peak signifies that covalent linkage appeared between the BAS and the carrier polymer after exposure to ionizing radiation and that a hyaluronidase - dextran 70 conjugate has been formed.

### Example 9

In 5 ml of a 20% Pluronic F-68 solution, dissolved was a 10-mg portion of recombinant proinsulin (from OAO National Biotechnologies, the town of Obolensk, Russia). A sample for HPLC was taken. Next, the solution was transferred to a polyethylene bag, sealed air tight, and frozen at -70°C for 16 hours. Following this, the bag was irradiated on an ILU-6 electron accelerator with a dose of 1.0 Mrad. From the irradiated bag, a sample was again taken for HPLC. Reversed-phase HPLC was carried out on a Sykam chromatograph system (Germany) using a Phenomenex Luna C18 5-µm 100A column (USA). The gradient mode was employed: phase A, 58.8%, by the 16th minute, 32.5%, by the 18th minute, 0%; phase B, 41.2%, by the 16 minute, 67.5%, by the 18th minute, 100%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 214 nm.

The results of HPLC are displayed in Fig. 15 and Fig. 16. The proinsulin peaks are peak 16 in Fig. 15 and peak 17 in Fig. 16.

After exposure to ionizing radiation, 96% of the proinsulin survived. The shift of the HPLC peak signifies that covalent linkage between the BAS and the carrier polymer appeared after exposure to ionizing radiation and that a proinsulin - Pluronic F-68 conjugate has been formed.

### Example 10

In 5 ml of a 20% Pluronic F-68 solution (this concentration is close to saturation), dissolved was a 50-mg portion of recombinant human insulin. A sample for HPLC was taken. Next, the solution was transferred to a polyethylene bag, sealed air tight, placed into a freezer maintained at -70°C, and kept there for 16 hours. Following this, the bag was irradiated on an ILU-6 electron accelerator with a dose of 1.5 Mrad. From the irradiated bag, a sample again was taken for HPLC. Reversed-phase HPLC was carried out on a Sykam chromatograph system (Germany) using a Phenomenex Luna C18 5-µm 100A column (USA). The isocratic mode was employed: phase A, 57.6%; phase B, 42.4%, wherein phase A was is 0.1 M Na₂SO₄ solution (pH 2.3) (90%) + acetonitrile (10%) and phase B is 0.1 Na₂SO₄ solution (pH 2.3) (50%) + acetonitrile (50%); detection was at 214 nm.

The results of HPLC are displayed in Fig. 17 and Fig. 18. The insulin peaks are peak 12 in Fig. 17 and peak 10 in Fig. 18.

After exposure to ionizing radiation, 50% of the insulin survived. The shift of the HPLC peak signifies that covalent linkage between the BAS and the carrier polymer appeared after exposure to ionizing radiation and that a proinsulin - Pluronic F-68 conjugate has been produced.

### Example 11

In 5 ml of a 20% polyethylene oxide-1500 solution, dissolved was a 50-mg portion of recombinant human insulin. A sample for HPLC was taken. Next, the solution was transferred to a polyethylene bag, sealed air tight, placed in a freezer maintained at -70°C, and kept there for 16 hours. Immediately before being irradiated, the bag was placed into a container with liquid nitrogen and kept there until its temperature reached -140°C. Immediately following this, the bag was irradiated on an ILU-6 electron accelerator with a dose of 5 Mrad. From the irradiated bag, a sample again was taken for HPLC. Reversed-phase HPLC was carried out on a Sykam chromatograph system (Germany) using a Phenomenex Luna C18 5-µm 100A column (USA). The isocratic mode was employed: phase A, 57.6%; phase B, 42.4%, wherein phase A was 90% 0.1 M Na₂SO₄ solution (pH 2.3) + 10% acetonitrile and phase B was 50% 0.1 Na₂SO₄ solution (pH 2.3) + 50% acetonitrile; detection was at 214 nm.

The results of HPLC are displayed in Fig. 19 and Fig. 20. The insulin peaks are peak 15 in Fig. 19 and peak 14 in Fig. 20.

After exposure to ionizing radiation, 59% of the insulin survived. The shift of the HPLC peak signifies that covalent linkage between the BAS and the carrier polymer appeared after exposure to ionizing radiation and that an insulin - polyethylene oxide-1500 conjugate has been formed.

### Example 12

An sample was prepared as follows: 2 mg of tyrosyl-2-alanyl-glycyl-phenylalanyl-leucyl-arginine diacetate was mixed with 1 ml of a 10% Plasdone K-17 solution. The solution was sealed in an air-tight polyethylene bag.

The sample contained in the polyethylene bag polyethylene bag was placed into a freezer maintained at -70°C and kept there for 16 hours, and afterwards was irradiated on an ILU-6 electron accelerator with a dose of 1.0 Mrad. Reversed-phase HPLC was carried out on a Sykam chromatograph system (Germany) a Phenomenex Luna C18 5-µm 100A column (USA). The isocratic mode was employed: phase A, 55%; phase B, 45%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 230 nm.

The results of HPLC are displayed in Fig. 21. The tyrosyl-2-alanyl-glycyl-phenylalanyl-leucyl-arginine diacetate peak is peak 8.

Against the sample exposed to ionizing radiation in a liquid form (Fig. 8), here the survival percentage of immobilized tyrosyl-2-alanyl-glycyl-phenylalanyl-leucyl-arginine diacetate is almost seven times as high. The shift of the tyrosyl-2-alanyl-glycyl-phenylalanyl-leucyl-arginine diacetate peak signifies that the BAS has been immobilized on the polymer and that a tyrosyl-2-alanyl-glycyl-phenylalanyl-leucyl-arginine diacetate - polyvinylpyrrolidone (Plasdone K-17) conjugate has been produced.

It follows that freezing of solutions before irradiation improved the survival of the polymer-immobilized BAS.

### Example 13

To 5 ml of a 1% polyethylene oxide-1500 solution, added was a 50-mg portion of recombinant human insulin (from OAO National Biotechnologies, the town of Obolensk (Russia)). Inasmuch as this grade of insulin is soluble in acid media and insoluble in neutral media such as polyethylene oxide solutions, glacial acetic acid was added to the mixture until pH was between 2.5 and 2.8 as monitored with a pH meter. As a result of acid titration, insulin dissolves in polyethylene oxide-1500 and insulin + polyethylene oxide mixtures in water become clear. Then, the solution was transferred to a polyethylene bag and sealed air tight. The bag was placed to a freezer maintained at -70°C and kept there for 12 hours. Following this, the bag was irradiated on an ILU-6 electron accelerator with a dose of 1.0 Mrad.

After irradiation, immobilized insulin did not precipitate when the solution was alkalinized to pH 9. In this way, evidence is obtained that covalent links appeared between the BAS and the carrier polymer and an insulin - polyethylene oxide-1500 conjugate has been formed.

### Example 14

In 5 ml of a 10% solution of dextran having a molecular mass of 40-60 kDa, dissolved was a 2.5-mg portion of somatotropin. A sample was taken for HPLC. Then, the solution was transferred to a polyethylene bag, sealed air tight, and frozen at -20°C for 16 hours. Following this, the bag was exposed to bremsstrahlung γ-rays in a dose of 0.5 Mrad. From the irradiated bag, a sample was taken for HPLC. Reversed-phase HPLC was carried out on a Sykam chromatograph system (Germany) using a Phenomenex Luna C18 5-µm 100A column (USA). The gradient mode was employed: phase A, 10%, by the 40th minute, 0%; phase B, 90%, by the 40th minute, 0%, wherein phase A was 0.1 M Na₂SO₄ solution (pH 2.3) (90%) + acetonitrile (10%) and phase B was 0.1 Na₂SO₄ solution (pH 2.3) (50%) + acetonitrile (50%); detection was at 220 nm.

The results of HPLC are displayed in Figs. 22 and 23. The somatotropin peaks are peak 4 in Fig. 22 and peak 3 in Fig. 23.

The percent survival of somatotropin after exposure to ionizing radiation was 93.5%. The shift of the HPLC peak signifies that covalent linkage between the BAS and the carrier polymer appeared upon exposure to ionizing radiation and that a somatotropin - dextran conjugate has been formed where dextran has a molecular mass of 40 to 60 kDa.

### Example 15

In 10 ml of a 10%-ΓO pacTBopa polyethylene oxide-1500, dissolved was a 100-mg portion of recombinant human insulin. A sample was taken for HPLC. Next, the solution was divided into three samples; the samples were transferred to polyethylene bags and sealed air tight.

Sample No. 1 was placed to a freezer which was maintained at -20°C and kept therein for 16 acoB. Samples No. 2 and No. 3 were placed into a freezer which was maintained at - 70°C and kept therein for 16 hours.

Once frozen, sample No. 1 was irradiated on an ILU-6 electron accelerator with a dose of 1 Mrad. Sample No. 2 was irradiated on an ILU-6 electron accelerator with a dose of 1.5 Mrad. Immediately before irradiation, the bag containing sample No. 3 was placed into a container with liquid nitrogen and kept therein until a temperature of -140°C was reached. Once this occurred, the bag was immediately irradiated on an ILU-6 electron accelerator with a dose of 5 Mrad.

From the irradiated bags, samples were again taken for HPLC. Reversed-phase HPLC was carried out on a Sykam chromatograph system (Germany) using a Phenomenex Luna C18 5-µm 100A column (USA). The isocratic mode was employed: phase A, 57.6%; phase B, 42.4%, wherein phase A was 0.1 M Na₂SO₄ solution (pH 2.3) (90%) + acetonitrile (10%) and phase B is 0.1 Na₂SO₄ solution (pH 2.3) (50%) + acetonitrile (50%); detection was at 214 nm.

The results of HPLC are displayed in Fig. 24, Fig. 25, Fig. 26, and Fig. 27. The insulin peaks are peak 19 in Fig. 24, peak 15 in Fig. 25, and peak 16 in Fig. 26 and Fig. 27.

In sample No. 1, 54% of the insulin survived; in sample No. 2, the percent survival was 41%; and in sample No. 3, 57%. The shift of HPLC peaks signifies that covalent link between the BAS and the carrier polymer appeared upon exposure to ionizing radiation and that an insulin - polyethylene oxide-1500 conjugate has been formed.

## Claims

1. A method for immobilization of a biologically active substance (BAS) on a pharmacologically acceptable water-soluble carrier polymer, comprising the dissolution of the BAS in a solution of said carrier and exposure of the reaction mixture to ionizing radiation, **characterized in that** the carrier polymer is used in concentrations ranging from above 10% to a saturated solution.

2. The method according to claim 1, wherein before being irradiated the reaction mixture is frozen to a temperature ranging from -20°C to -140°C.

3. The method according to claim 1 or 2, wherein the BAS is a substance selected from the group comprising insulin, proinsulin, gCSF, hyaluronidase, tyrosyl-2-alanyl-glycyl-phenylalanyl-leucyl-arginine diacetate, alcalase, and somatotropin.

4. The method according to claim 1, wherein the carrier polymer is a polymer selected from the group comprising polyethylene oxide, Pluronic, dextran, polyvinylpyrrolidone, and acrylamide.

5. The method according to claim 1, wherein the ionizing radiation is an accelerated electron beam or bremsstrahlung γ-rays in a dose of 0.5 to 5 Mrad.

6. The method according to claim 1, wherein alcalase is immobilized on polyethylene oxide.

7. The method according to claim 1 or 2, wherein insulin is immobilized on polyethylene oxide.

8. The method according to claim 1 or 2, wherein tyrosyl-2-alanyl-glycyl-phenylalanyl-leucyl-arginine diacetate is immobilized on polyvinylpyrrolidone.

9. The method according to claim 2, wherein granulocyte colony stimulating factor is immobilized on polyethylene oxide.

10. The method according to claim 2, wherein hyaluronidase is immobilized on dextran.

11. The method according to claim 2, wherein proinsulin is immobilized on Pluronic.

12. The method according to claim 2, wherein insulin is immobilized on Pluronic.

13. A method of immobilization of a BAS on a pharmacologically acceptable water-soluble polymer, comprising the dissolution of the BAS in a solution of a said carrier and exposure of the reaction mixture to ionizing radiation, **characterized in that** before being irradiated, the mixture is frozen to a temperature ranging from -20°C to 140°C.

14. The method according to claim 13, wherein the pharmacologically acceptable water-soluble polymer is used in concentrations ranging from above 0% to a saturated solution.

15. The method according to claim 13, wherein the BAS is a substance selected from the group comprising insulin, proinsulin, gCSF, hyaluronidase, tyrosyl-2-alanyl-glycyl-phenylalanyl-leucyl-arginine diacetate, alcalase, and somatotropin.

16. The method according to claim 13, wherein the carrier polymer is a polymer selected from the group comprising polyethylene oxide, Pluronic, dextran, polyvinylpyrrolidone, and acrylamide.

17. The method according to claim 13, wherein the ionizing radiation is an accelerated electron beam or bremsstrahlung γ-rays in a dose of 0.5 to 5 Mrad.

18. The method according to claim 13, wherein tyrosyl-2-alanyl-glycyl-phenylalanyl-leucyl-arginine diacetate is immobilized on polyvinylpyrrolidone.

19. The method according to claim 13, wherein insulin is immobilized on polyethylene oxide.

20. The method according to claim 13, wherein somatotropin is immobilized on dextran.

21. The method according to claim 13, wherein proinsulin is immobilized on acrylamide.

22. The method according to claim 13, wherein granulocyte colony stimulating factor is immobilized on polyethylene oxide.

23. The method according to claim 13, wherein hyaluronidase is immobilized on dextran.

24. The method according to claim 13, wherein insulin is immobilized on Pluronic.

25. A BAS - carrier conjugate, **characterized in that** the BAS is selected from the group comprising of proinsulin, tyrosyl-2-alanyl-glycyl-phenylalanyl-leucyl-arginine diacetate, alcalase, hyaluronidase, and somatotropin and **in that** the carrier is a pharmacologically acceptable water-soluble polymer which is selected from the group comprising polyethylene oxide, polyvinylpyrrolidone, dextran, Pluronic, and acrylamide, wherein a BAS molecule is covalently linked to at least one polymer molecule.

26. The conjugate according to claim 25, wherein the BAS is tyrosyl-2-alanyl-glycyl-phenylalanyl-leucyl-arginine and the polymer is polyvinylpyrrolidone (Plasdone K-17).

27. The conjugate according to claim 25, wherein the BAS is alcalase and the polymer is polyethylene oxide having a molecular mass of 300 Da.

28. The conjugate according to claim 25, wherein the BAS is hyaluronidase and the polymer is dextran having a molecular mass of 70 kDa.

29. The conjugate according to claim 25, wherein the BAS is proinsulin and the polymer is Pluronic F-68.

30. The conjugate according to claim 25, wherein the BAS is somatotropin and the polymer is dextran having a molecular mass of 40-60 kDa.

31. The conjugate according to claim 25, wherein the BAS is proinsulin and the polymer is acrylamide 4K.
